# EUROPÄISCHE PATENTSCHRIFT

(11) **EP 2 615 269 B1**
(45) Veröffentlichungstag und Bekanntmachung des Hinweises auf die Patenterteilung: **13.08.2014**
(21) Anmeldenummer: 12151046.5
(22) Anmeldetag: 13.01.2012
(51) Int. Cl.: F01M 11/10, G01N 21/59, F01M 13/02

(54) **Vorrichtung und Verfahren zum Ermitteln von Messwerten von Gasen und/oder eines Aerosols für eine Arbeitsmaschine**
Device and method for determining the parameters of gases and/or an aerosol for a work machine
Dispositif et procédé de détermination de valeurs de mesure de gaz et/ou d'un aérosol pour une machine de travail

(43) Veröffentlichungstag der Anmeldung: 17.07.2013
(73) Patentinhaber: Schaller Automation Industrielle Automationstechnik GmbH & Co. KG, 66440 Blieskastel (DE)
(72) Erfinder: Gnauert, Uwe, Dr., 66440 Blieskastel (DE)
(74) Vertreter: Schmauder & Partner AG Patent- & Markenanwälte VSP

(56) Entgegenhaltungen:
- WO-A2-2007/140640
- DE-A1- 2 608 390
- JP-A- 7 310 519
- JP-A- 10 115 210

## Beschreibung

### Technisches Gebiet

Die Erfindung betrifft eine Anlage mit Messvorrichtungen zum Ermitteln von Messwerten von Gasen und/oder eines Aerosols für eine Arbeitsmaschine, gemäss dem Oberbegriff von Anspruch 1, und ein Überwachungsverfahren, bei dem Messwerte von Gasen und/oder eines Aerosols in einer Arbeitsmaschine, insbesondere Messwerte der Aerosolkonzentration in einer Brennkraftmaschine, ermittelt werden.

### Stand der Technik

Die Überwachung von Gasen und Aerosolkonzentrationen, insbesondere von Schmierölnebel in Arbeitsräumen von Brennkraftmaschinen, von Kraftstoff-Hochdruckeinheiten zur Versorgung von Brennkraftmaschinen oder von Leistungsgetrieben, ist zur Vermeidung von Schäden von erheblicher Bedeutung. Ein schneller Anstieg der Ölnebelkonzentration lässt nämlich auf Schäden, beispielsweise das Reissen eines Schmierfilms, schliessen. In Folge der dabei entstehenden Reibungswärme bilden sich Öldämpfe, die im Arbeitsraum zu Ölnebel kondensieren und so zu einem schnellen Anstieg der Ölnebelkonzentration führen. Wenn die daraus resultierende Gefahr schnell erkannt wird, können durch entsprechende Gegenmassnahmen wie z.B. ein Stilllegen der Maschine oder von einzelnen Maschinenkomponenten Explosionen und somit Gefahren für Personen und weitere Schäden an der Maschine vermieden werden. Es ist aber auch möglich, durch andere Sensoren bestimmte Gasanteile in einer solchen Arbeitsatmosphäre zu untersuchen.

Weiterhin können neben dem Abreissen des Schmierfilms in Lagern bei Kolbenmotoren zwischen dem Kolben und seiner Zylinderwand infolge schadhafter Kolbenringe - sogenannte Durchbläser - auftreten, die zu einer totalen Beschädigung des Kolben-/Zylinder-Aggregates führen ("Kolbenfresser"). Eine Erhöhung der Ölnebeldichte bei gleichzeitigem Temperaturanstieg in Folge der heissen Verbrennungsgase lassen auf einen solchen Durchbläser schliessen.

Erste Ansätze zur Messung der Ölnebelkonzentration sind aus der EP-A-0 071 391 bekannt. In der EP-A-0 071 391 wird vorgeschlagen, mit Hilfe eines Gebläses das Aerosol aus dem Arbeitsraum durch eine Messkammer zu saugen und dort eine Reflektionsmessung mit Hilfe einer Strahlungsquelle und eines Strahlungssensors durchzuführen. Das dort vorgeschlagene Flügelradgebläse soll für eine Vielzahl von parallel angeordneten Kammern verwendet werden.

Schon in der WO-A-98/11331 wurden die Nachteile einer solchen Anordnung aufgezeigt. Neben dem erheblichen konstruktiven und betrieblichen Aufwand für die Anordnung hat sich der Einsatz eines Gebläses zum Absaugen als mangelhaft herausgestellt, so dass eine solche Lösung vermieden werden sollte. Durch das Absaugen wird überdies Schmutzluft durch das Rohrsystem gesaugt, dabei können Ölansammlungen in Form von Ölsäcken auftreten, die die Rohrleitungen verstopfen und die Funktion der Messvorrichtung erschweren und verunmöglichen.

Dagegen wird in der DD-A-239 474 und in der GB-A-2 166 232 vorgeschlagen, für jeden Arbeitsraum des Triebwerkes einer Brennkraftmaschine eine Sensoreinheit direkt im Inneren des jeweiligen Arbeitsraumes anzuordnen und über eine optische oder elektrische Übertragungsstrecke mit einer ausserhalb der Brennkraftmaschine befindlichen Auswerteeinheit zu verbinden. Eine solche Lösung ist jedoch mit dem Nachteil verbunden, dass die Basiskonzentration an Ölnebel und Spritzöl auf Dauer die Sensoren verschmutzen und somit auch zu Fehlalarmen führt.

In der bereits genannten WO-A-98/11331A wird dagegen vorgeschlagen, in jedem zu überwachenden Arbeitsraum eine Sensoreinheit mit einer Absaugung mittels einer Venturidüse vorzusehen. Eine solche Messvorrichtung arbeitet ohne mechanisch bewegte Teile und ist somit nahezu verschleissfrei.

Ähnliches ist aus der JP 7/310519 A bekannt. Daraus und zuvor schon aus der DE 26 08 390 A1 ist es auch bereits bekannt, dass eine optische Messstrecke, die einen optischen Sender und einen optischen Empfänger aufweist, mit einem so genannten Vorhang versehen wird, mit dem die genannten optischen Elemente möglichst frei von Ölnebelniederschlägen etc. gehalten werden. Bislang wurde dies so verwirklicht, dass ein Frischluftstrom senkrecht zur optischen Messstrecke jeweils in der Nähe der optischen Elemente erzeugt wurde. Dies ist jedoch aufwändig gemessen an der Wirkung.

Nachteilig an der Vorrichtung nach der WO-A-98/11331A hat sich weiterhin herausgestellt, dass der Energieaufwand der Venturipumpe zum Ansaugen des Aerosolgemisches aus dem Triebraum durch die optische Messstrecke hindurch sehr hoch ist, insbesondere wenn die optische Messstrecke in einem Gehäuse untergebracht ist, die mit einer Leitung mit der Venturipumpe verbunden ist.

### Darstellung der Erfindung

Aufgabe der Erfindung ist es, eine verbesserte Anlage mit Messvorrichtungen der eingangs genannten Art vorzuschlagen zum Ermitteln von Messwerten eines Gases und/oder Aerosols für eine Arbeitsmaschine. Insbesondere soll der Aufwand und hier insbesondere der Energieaufwand kleiner gehalten werden als beim Stand der Technik.

Diese Aufgabe der Erfindung wird durch eine Messvorrichtung nach Anspruch 1 gelöst. Dabei haben die Massnahmen der Erfindung zunächst einmal zur Folge, dass der notwendige Druckluftstrom gegenüber dem Stand der Technik erheblich verringert ist. Ein besonderer Vorteil der genannten Massnahmen der Erfindung ist aber die Möglichkeit einer sehr kompakten Bauweise. Weiterhin hat sich herausgestellt, dass durch die Massnahmen der Erfindung wesentlich bessere Ergebnisse erzielt werden können als mit den Messvorrichtungen gemäss dem Stand der Technik.

Vorteilhaft ist es, wenn die optische Sensoreinheit so eingerichtet ist, dass der optische Durchgang zwischen dem optischen Emitter und dem optischen Empfänger seitlich versetzt zur Austrittsrichtung der Druckluftdüse angeordnet ist. In diesem Fall wird eine optimale Menge an abgesaugten Triebraumgas (Aerosolgemisch) mit dem optischen Strahl erfasst, während die Hauptmenge an durchgehender Druckluft, die ja nur zum Antrieb, also zum Absaugen des Triebraumgases eingesetzt wird, an der Messstrecke vorbei geleitet wird, ohne nennenswerten Einfluss auf die Messung zu nehmen.

Um die optischen Elemente frei von Ölnebelablagerungen zu halten, ist es vorteilhaft wenn die Sperrluftdüsen so angeordnet und eingerichtet sind, dass ein weiterer Luftstrom jeweils vom optischen Emitter und vom optischen Empfänger zum Mischraum von Druckluft und Absaugatmosphäre ermöglicht wird. Damit wird ein Gegenstrom zur möglichen unerwünschten Diffusionsrichtung des Ölnebels erzeugt. Dieser Gegenstrom ist ohne grossen Aufwand ausreichend, um jegliche Ablagerung auf den optischen Elementen zu vermeiden. Mit dieser Massnahme kann auf den aufwändigen "Vorhang" verzichtet werden. Die Sperrluftdüsen und damit der genannte Gegenstrom können allenfalls alleine dadurch betrieben werden, dass die Druckluftzufuhr von der Druckluftdüse diese Strömung mit betreibt. Üblicherweise werden die Sperrluftdüsen aber mit einer Abzweigung von der Druckleitung betrieben.

Besonders vorteilhaft ist die Messvorrichtung gemäss der vorliegenden Erfindung, wenn die Absaugung aus dem Motorraum vorzugsweise mittels einer Ventileinrichtung unterbrochen und durch eine Frischluftzufuhr ersetzt werden kann. In diesem Fall kann eine Absolutmessung durchgeführt werden, indem das Ventil - vorzugsweise ein Magnetventil vor der Venturidüse geschlossen wird und eine Spülung des Messkanals mit Frischluft erfolgt.

Ein zweiter Aspekt der vorliegenden Erfindung betrifft die Durchführung der Messung mit der vorliegenden Messvorrichtung.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Weitere Einzelheiten, Vorteile und Merkmale des Gegenstandes der vorliegenden Erfindung ergeben sich aus der nachfolgenden Beschreibung der dazu gehörenden Zeichnungen, in denen - beispielhaft - erfindungsgemässe Messvorrichtungen erläutert werden. In den Zeichnungen zeigt:
- Figur 1: eine Messvorrichtung in schematischer, perspektivischer Darstellung am Arbeitsraum einer Brennkraftmaschine, gemäss einem ersten Ausführungsbeispiel der vorliegenden Erfindung und
- Figur 2: eine Darstellung der Schaltelemente der Druckversorgung in der Messvorrichtung nach Figur 1.

### Wege zur Ausführung der Erfindung

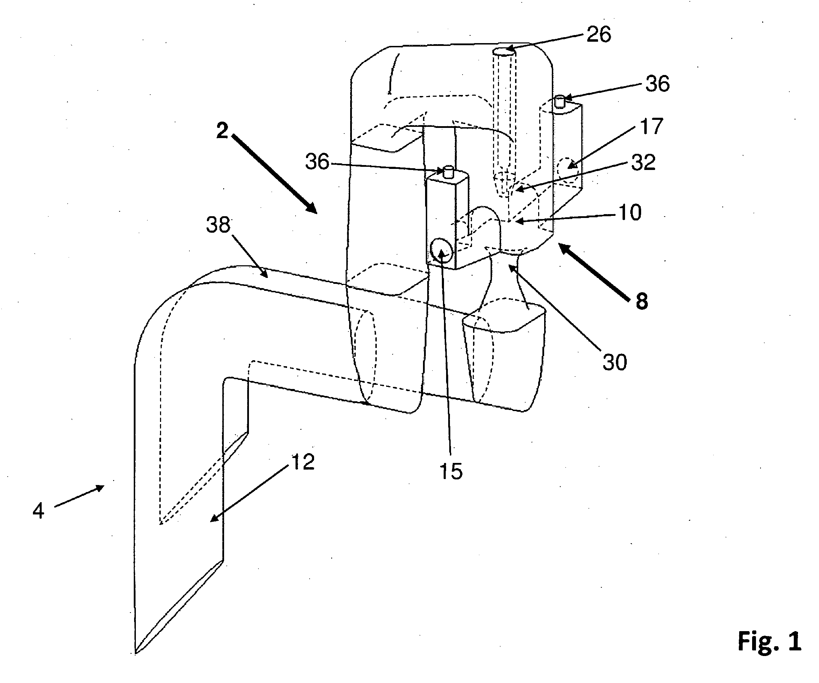

Die Figur 1 zeigt eine Messvorrichtung 2, die mit ihrem Gehäuse lösbar an einem Arbeitsraum 4 einer Arbeitsmaschine angeordnet ist. Im vorliegenden Beispiel ist die Arbeitsmaschine eine Brennkraftmaschine wie ein Hubkolbenmotor, beispielsweise ein Dieselmotor. Die Messvorrichtung dient zum Ermitteln von Messwerten eines Aerosols, hier des Ölnebels im Arbeitsraum der Arbeitsmaschine. Die Messvorrichtung 2 enthält als Saugmittel eine Druckluftstrahlpumpe 8, deren Unterdruckbereich 10 über eine Saugleitung 12 über Öffnung in der Wand des Arbeitsraumes mit dem Inneren des Arbeitsraumes 4 in Verbindung steht. Senkrecht zur Austrittsrichtung der Druckluftdüse 32 und damit auch im Wesentlichen senkrecht zur Achse zwischen Druckluftdüse 32 und der Mitte des Trichters 30 ist ein Lichtkanal ausgebildet , an dessen einen Ende ein Lichtemitter 15 und an dessen anderen Ende ein Lichtempfänger 17 einer Sensoreinheit zur Ermittlung der Messwerte des Ölnebels angeordnet sind. Die Sensoreinheit umfasst ein Elektronikmodul. Im vorliegenden Ausführungsbeispiel ist der Lichtstrahl des Lichtemitters 15 auf den Lichtempfänger 17 gerichtet, wobei der Lichtstrahl durch den Unterdruckbereich der Druckstrahlpumpe hindurchgeht, senkrecht aber seitlich etwas versetzt zur Austrittsrichtung der Druckstrahldüse und damit auch im Wesentlichen senkrecht zur Achse zwischen Druckluftdüse 32 und der Mitte des Trichters 30.

Die Druckluftstrahlpumpe 8 ist mit einer Druckluftzufuhr 26 wirkverbunden, die in der Druckluftdüse 32 mündet, welche den Druckluftstrahl in einen Trichter 30 einbläst und dadurch in dem den Übergangsbereich zwischen der Druckluftdüse 32 und dem Trichter 30 umgebenden Unterdruckbereich 10 einen Unterdruck erzeugt. Die Saugleitung 12 ist mit dem Unterdruckbereich 10 verbunden. Die Austrittsrichtung der Druckluftdüse 32 ist im vorliegenden Ausführungsbeispiel in etwa in die Mitte des Trichters 30 gerichtet.

An der Druckluftzufuhr 26 ist eine Abzweigleitung 106 angeschlossen, die an den Sperrluftdüsen 36 münden. Die eine Sperrluftdüse ist in der Nähe des Lichtemitters 15 und die andere Sperrluftdüse ist in der Nähe des Lichtempfängers angeordnet und an beiden Sperrluftdüsen 36 tritt Druckluft aus, die zu der aus Druckluftdüse 32 und Trichter 30 bestehenden Venturianordnung fliesst und - zusammen mit der aus der Druckluftdüse 32 austretenden Druckluft - mittels des als Rückführung in den Motorraum ausgebildeten Rückführungskanal 38 zurückgeführt wird. Durch diese Anordnung wird der aus der Saugleitung 12 allenfalls austretende Ölnebel wirksam daran gehindert, zum Lichtemitter 15 oder zum Lichtempfänger 17 zu gelangen und diese zu verschmutzen. Eine solche Anordnung hat sich als wirksamer erwiesen als die vormals bekannte "Vorhanglösung".

Es sollte an dieser Stelle bemerkt werden, dass die optische Anordnung, die im vorliegenden Ausführungsbeispiel mit einem an einem Ende des Lichtkanals angeordneten Lichtemitter 15 und einem am anderen Ende des Lichtkanals angeordneten Lichtempfänger 17 ausgebildet ist, praktische gleichwirkend ausgebildet sein kann, indem sowohl der Lichtemitter 15 als auch der Lichtempfänger 17 an einem Ende des Lichtkanals angeordnet sind und am anderen Ende des Lichtkanals ein Reflektor (z.B. ein Spiegel) angeordnet ist, der so ausgerichtet ist, dass der Lichtstrahl vom Lichtemitter 15 auf den Lichtempfänger 17 reflektiert wird, vorzugsweise jeweils durch den Unterdruckbereich hindurch und senkrecht aber seitlich etwas versetzt zur Austrittsrichtung der Druckstrahldüse und damit auch im Wesentlichen senkrecht zur Achse zwischen Druckluftdüse 32 und der Mitte des Trichters 30.

Weiterhin sollte darauf hingewiesen werden, dass im vorliegenden Ausführungsbeispiel zwar ein Lichtstrahl im Frequenzbereich des nahen Infrarot (860 nm) zum Einsatz vorgesehen ist, dass aber auch Wellenlängen im gesamten Spektrum von Infrarot bis Ultraviolett oder eine Mischung davon zum Einsatz kommen kann.

Das Elektronikmodul der Sensoreinheit ist beispielsweise über eine Leitung mit einer externen Auswerteinrichtung verbunden, welche die gemessene Ölnebelkonzentration anzeigt. Übersteigt die Ölnebelkonzentration einen vorbestimmbaren Schwellwert, der auf einen Defekt in der Arbeitsmaschine hindeutet, so kann ein entsprechendes Alarmsignal ausgegeben werden oder die Arbeitsmaschine abgestellt werden.

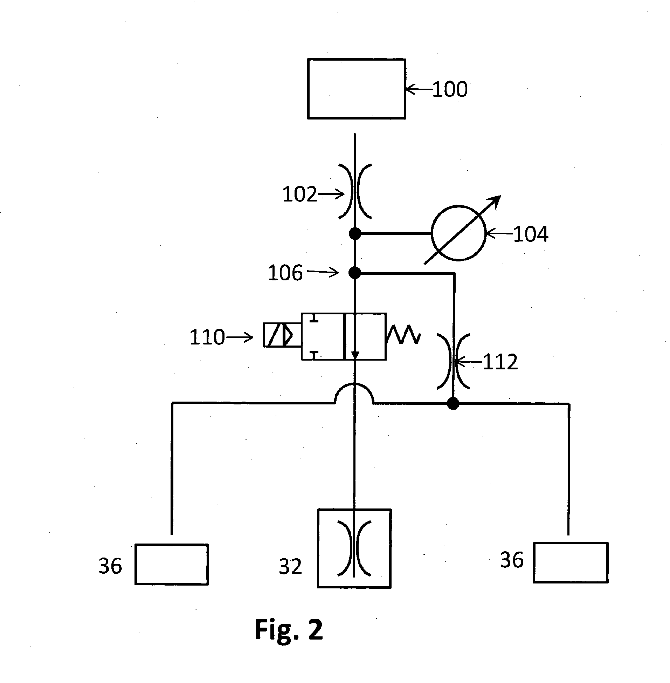

Die Druckversorgung des vorliegenden Ausführungsbeispiels ist in Figur 2 dargestellt. Die Druckversorgung 100, mit im vorliegenden Ausführungsbeispiel einen Druck von 3,0 bar, stellt mittels der Hauptdrossel 102, mit einem Durchmesser von 022 mm, einen Volumenstrom von 100l/h zur Verfügung. Ein Drucksensor 104 ist zur Überwachung der Druckversorgung und damit der sich einstellenden Volumenströme durch die Druckluftdüse 32 und die Sperrluftdüsen 36 zwischen der Hauptdrossel 102 und dem Magnetventil 11 bzw. der Sperrluftdrossel 122 angeordnet. Der Hauptzweig wird über ein Magnetventil 110 - im Ausführungsbeispiel mit einem Durchmesser von 0.45 mm zur Druckluftdüse 32 geführt, wobei ein Volumenstrom von 66 l/h der Druckluftdüse 32 zur Verfügung steht. Der Sperrluftzweig wird über eine Sperrluftdrossel 112 mit einem Durchmesser von 0.26 mm gleichmässig zu den beiden Sperrluftdüsen 36 geführt, denen jeweils ein Luftstrom von 17l/h zur Verfügung steht.

Im Normalbetrieb strömt bei der Messvorrichtung des vorstehend beschriebenen Ausführungsbeispiels als Druckluft von der Druckluftversorgung 100 durch die Hauptdrossel 102 sowohl durch das geöffnete Magnetventil 110 durch die Druckluftdüse 32 und erzeugt dabei einen die Motoratmosphäre mitreissenden Unterdruck als auch durch die Sperrdrossel 112 durch die Sperrluftdüsen 36 und erzeugt damit einen die optischen Elemente 15 und 17 schützenden Gegenstrom von den optischen Elementen zum Trichter 30.

Im Kalibrationsbetrieb dagegen ist das Magnetventil 110 geschlossen und der Hauptstrom durch die Druckluftdüse 32 ist unterbrochen. Der optische Kanal zwischen den optischen Elementen 15 und 17 wird durch die Sperrluftdüsen 36 gereinigt und die Messvorrichtung kann einer absoluten Messung des Lichtdurchgangs unterworfen werden. Dieser Vorgang kann beispielsweise einmal am Tag vorgesehen werden und ermöglicht die Einstellung des oben genannten Schwellwertes, da die Messung damit unabhängig von der Emmissionsleistung des Emitterelementes 15 und der Sensitivität des Lichtempfängers 17 und auch weiterer Störungen gestaltet werden kann.

Es sollte darauf hingewiesen werden, dass die vorstehend beschriebene Anordnung durch den Drucksensor 104 in der Lage ist, die Funktionsfähigkeit aller der genannten Düsen zu überwachen, indem der absolute Druck gemessen wird und damit Fehler sowohl in einer der Sperrluftdüsen 36 als auch in beiden Sperrluftdüsen - durch Vergleich mit dem Solldruck der jeweiligen Konfiguration - leicht festgestellt werden kann.

### Bezugszeichenliste

- 2: Messvorrichtung zum Ermitteln von Messwerten für ein Aerosol im
- 4: Arbeitsraum
- 8: Saugmittel/ Druckluftstrahlpumpe
- 10: Unterdruckbereich
- 12: Saugleitung
- 15: optischer Emitter
- 17: optischer Empfänger
- 26: Druckluftzufuhr
- 30: Trichter
- 32: Druckluftdüse
- 36: Sperrluftdüsen
- 38: Rückführungskanal
- 100: Druckversorgung
- 102: Hauptdrossel
- 104: Drucksensor
- 106: Abzweigung
- 110: Magnetventil
- 112: Sperrluftdrossel

## Patentansprüche

1. Messvorrichtung (2) zum Ermitteln von Messwerten für ein Aerosol im Arbeitsraum (4) einer Arbeitsmaschine, nämlich der Aerosolkonzentration im Arbeitsraum einer Brennkraftmaschine, mit einem Saugmittel (8), das ein Aerosol-Luftgemisch aus dem Arbeitsraum (4) der Arbeitsmaschine absaugt und einer optischen Sensoreinheit mit zumindest einem optischen Emitter (15) und zumindest einem optischen Empfänger (17) zuführt, und mit einer Elektronikeinrichtung für den Betrieb der optischen Sensoreinheit, wobei das Saugmittel als Druckluftstrahlpumpe (8) mit einer Druckluftzufuhr (26), einer Druckluftdüse (32), einemvorzugsweise trichterförmigen Druckableitungskanal (30) und einem Unterdruckbereich (10) ausgebildet ist, die Druckluftdüse (32) eine Austrittsrichtung hat, die im Wesentlichen in Richtung des Druckableitungskanal gerichtet ist, die Druckluftzufuhr (26) mit einer Druckluftquelle verbindbar ist und am Unterdruckbereich (10) eine mit dem Arbeitsraum (4) der Arbeitsmaschine verbindbare Saugleitung (12) angeschlossen ist,
**dadurch gekennzeichnet, dass**
die optische Sensoreinheit einen optischen Durchgang zwischen dem optischen Emitter (15) und dem optischen Empfänger (17) aufweist, der im Wesentlichen senkrecht zur Austrittsrichtung der Druckluftdüse (32) ausgerichtet ist, und durch den Unterdruckbereich hindurchführt.

2. Messvorrichtung nach Anspruch 1, **dadurch gekennzeichnet, dass** die optische Sensoreinheit so eingerichtet ist, dass der optische Durchgang zwischen dem optischen Emitter (15) und dem optischen Empfänger (17) seitlich versetzt zur Austrittsrichtung der Druckluftdüse (32) angeordnet ist.

3. Messvorrichtung (2) nach Anspruch 1 oder 2, **gekennzeichnet durch** Sperrluftdüsen (36), die so angeordnet und eingerichtet sind, dass ein weiterer Luftstrom jeweils vom optischen Emitter und vom optischen Empfänger zum Unterdruckbereich ermöglicht wird.

4. Messvorrichtung nach Anspruch 3, **dadurch gekennzeichnet, dass** von der Druckluftzufuhr (26) jeweils eine Abzweigleitung zu den Sperrluftdüsen (36) aufweist.

5. Messvorrichtung nach einem der Ansprüche 1 bis 4, **dadurch gekennzeichnet, dass** die Absaugung aus dem Motorraum vorzugsweise mittels einer Ventileinrichtung unterbrochen und durch eine Frischluftzufuhr ersetzt werden kann.

6. Verfahren zum Ermitteln von Messwerten für ein Aerosol im Arbeitsraum (4) einer Arbeitsmaschine, nämlich der Aerosolkonzentration im Arbeitsraum einer Brennkraftmaschine, mittels einer Messvorrichtung nach einem der Ansprüche 1 bis 5, wobei ein Aerosol-Luftgemisch aus dem Arbeitsraum (4) der Arbeitsmaschine absaugt und der optischen Sensoreinheit mit zumindest einem optischen Emitter und zumindest einem optischen Empfänger zuführt wird , und mit einem Elektronikmodul für den Betrieb der optischen Sensoreinheit, die Druckluftzufuhr (26) mit einer Druckluftquelle verbunden ist und am Unterdruckbereich (10) eine mit dem Arbeitsraum (4) der Arbeitsmaschine verbundene Saugleitung (12) angeschlossen ist,
**dadurch gekennzeichnet, dass**
mittels der optischen Sensoreinheit ein Lichtstahl im Frequenzbereich im unter oder oberhalb des sichtbaren Bereichs oder mit einer Mischung daraus vom optischen Emitter (15) zum optischen Empfänger (17) ausgesandt wird, wobei der Lichtstrahl im Wesentlichen senkrecht zur Austrittsrichtung der Druckluftdüse (32) ausgerichtet ist, und durch den Unterdruckbereich(10) hindurchgeführt wird.

7. Verfahren nach Anspruch 6, **dadurch gekennzeichnet, dass** der Lichtstrahl zwischen dem optischen Emitter (15) und dem optischen Empfänger (17) seitlich versetzt zur Austrittsrichtung der Druckluftdüse (32) ausgesendet wird.

8. Verfahren nach Anspruch 6 oder 7, **dadurch gekennzeichnet, dass** ein weiterer Luftstrom mittels Sperrluftdüsen (36) jeweils vom optischen Emitter und vom optischen Empfänger zum Unterdruckbereich geführt wird, wobei vorzugsweise Druckluft jeweils von einer Abzweigleitung zu den Sperrluftdüsen (36) geführt wird.

## Claims

1. A measuring device (2) for determining measured data for an aerosol in the working chamber (4) of a machine, namely the aerosol concentration in the working chamber of an internal combustion engine, with a suction means (8) that draws out an aerosol air mixture from the working chamber (4) of the machine and feeds the same to an optical sensor unit having at least an optical emitter (15) and at least an optical receiver (17), and with an electronic device for operating the optical sensor unit, wherein the suction means is configured as a compressed air jet pump (8) with a compressed air feed (26), a compressed air nozzle (32), a preferably funnel-shaped pressure discharge channel (30) and an underpressure region (10), the compressed air nozzle (32) having an outlet direction which is oriented substantially in the direction of the pressure discharge channel, the compressed air feed (26) being connectable to a compressed air source, and a suction line (12) that is connectable to the working chamber (4) of the machine being connected to the underpressure region (10),
**characterized in that**
the optical sensor unit has an optical passage between the optical emitter (15) and the optical receiver (17) which is oriented substantially perpendicular to the outlet direction of the compressed air nozzle (32) and leads through the underpressure region.

2. The measuring device according to claim 1, **characterized in that** the optical sensor unit is configured in such manner that the optical passage between the optical emitter (15) and the optical receiver (17) is laterally displaced with respect to the outlet direction of the compressed air nozzle (32).

3. The measuring device (2) according to claim 1 or 2, **characterized by** sealing air nozzles (36) that are arranged and configured in such manner as to allow for a further air flow each from a respective optical emitter and from a respective optical receiver towards the underpressure region.

4. The measuring device according to claim 3, **characterized in** comprising a respective junction line from the compressed air feed (26) to the sealing air nozzles (36).

5. The measuring device according to one of claims 1 to 4, **characterized in that** the suction from the engine compartment can be interrupted and replaced by a fresh air feed, preferably by means of a valve device.

6. A method for determining measured data for an aerosol in the working chamber (4) of a machine, namely the aerosol concentration in the working chamber of an internal combustion engine, by means of a measuring device according to one of claims 1 to 5, wherein an aerosol air mixture is drawn out from the working chamber (4) of the machine and fed to the optical sensor unit having at least an optical emitter and at least an optical receiver, and which has an electronic module for operating the optical sensor unit, the compressed air feed (26) being connected to a compressed air source, and a suction line (12) connected to the working chamber (4) of the machine being connected to the underpressure region (10),
**characterized in that**
a light beam in the frequency range within, below or above the visible range, or with a mixture thereof, is emitted, by means of the optical sensor unit, from the optical emitter (15) to the optical receiver (17), the light beam being oriented substantially perpendicular to the outlet direction of the compressed air nozzle (32), and being guided through the underpressure region.

7. The method according to claim 6, **characterized in that** the light beam between the optical emitter (15) and the optical receiver (17) is emitted laterally displaced with respect to the outlet direction of the compressed air nozzle (32).

8. The method according to claim 6 or 7, **characterized in that** a further air flow is led by means of sealing air nozzles (36) from a respective optical emitter and from a respective optical receiver to the underpressure region, wherein preferably compressed air is guided from a respective junction line to the sealing air nozzles (36).

## Revendications

1. Dispositif de mesure (2) permettant de détecter des valeurs de mesure d'un aérosol dans la chambre de travail (4) d'une machine de travail, à savoir la concentration en aérosol dans la chambre de travail d'un moteur à combustion interne, comportant des moyens d'aspiration (8) qui aspirent un mélange aérosol-air dans la chambre de travail (4) de la machine de travail et le transfère dans une unité de capteur optique comprenant au moins un émetteur optique (15) et au moins un récepteur optique (17), ainsi qu'un dispositif électronique pour commander le fonctionnement de l'unité de capteur optique, les moyens d'aspiration étant réalisés sous la forme d'une pompe à jet d'air comprimé (8) comprenant une alimentation en air comprimé (26) une buse d'air comprimé (32) un canal de décharge de pression (30) de préférence en forme d'entonnoir et une zone basse pression (10), la buse d'air comprimé (32) ayant une direction de sortie qui est essentiellement orientée vers le canal de décharge de pression, l'alimentation en air comprimé (26) pouvant être reliée à une source d'air comprimé, et dans la zone basse pression (10) étant montée une conduite d'aspiration (12) pouvant être reliée à la chambre de travail (4) de la machine de travail,
**caractérisé en ce que**
l'unité de capteur optique comporte un passage optique entre l'émetteur optique (15) et le récepteur optique (17) qui est essentiellement dirigé perpendiculairement à la direction de sortie de la buse d'air comprimé (32) et passe au travers de la zone basse-pression.

2. Dispositif de mesure conforme à la revendication 1,
**caractérisé en ce que**
l'unité de capteur optique est conçue de sorte que le passage optique entre l'émetteur optique (15) et le récepteur optique (17) soit décalé latéralement par rapport à la direction de sortie de la buse d'air comprimé (32).

3. Dispositif de mesure (2) conforme à la revendication 1 ou 2,
**caractérisé par**
des buses d'air de soufflage (36) qui sont montées et orientées de façon à permettre le passage d'un autre flux d'air respectivement de l'émetteur optique et du récepteur optique vers la zone basse pression.

4. Dispositif de mesure conforme à la revendication 3,
**caractérisé en ce que**
de l'alimentation en air comprimé (26) une conduite de dérivation conduit respectivement vers les buses d'air soufflage (36).

5. Dispositif de mesure conforme à l'une des revendications 1 à 4,
**caractérisé en ce que**
l'aspiration dans la chambre du moteur peut être interrompue de préférence au moyen d'un dispositif de soupape et remplacée par une alimentation en air frais.

6. Procédé permettant de détecter des valeurs de mesure d'un aérosol dans la chambre de travail (4) d'une machine de travail, à savoir la concentration en aérosol dans la chambre de travail d'un moteur à combustion interne au moyen d'un dispositif de mesure conforme à l'une des revendications 1 à 5, selon lequel un mélange aérosol/air est aspiré dans la chambre de travail (4) de la machine de travail et transféré à l'unité de capteur optique comprenant au moins un émetteur optique et au moins un récepteur optique et un module électronique pour commander le fonctionnement de l'unité de capteur optique, l'alimentation en air comprimé (26) étant reliée à une source d'air comprimé, et, dans la zone basse pression (10) étant montée une conduite d'aspiration (12) reliée à la chambre de travail (4) de la machine de travail,
**caractérisé en ce que**
l'unité de capteur optique émet un faisceau lumineux dans des plages de fréquences situées en-dessous ou au-dessus du domaine visible ou dans un mélange de ces plages, de l'émetteur optique (15) vers le récepteur optique (17), ce faisceau lumineux étant essentiellement orienté perpendiculairement à la direction de sortie de la buse d'air comprimé (32), et passant au travers de la zone basse-pression (10).

7. Procédé conforme à la revendication 6,
**caractérisé en ce que**
le faisceau lumineux entre l'émetteur optique (15) et le récepteur optique (17) est émis en étant latéralement décalé par rapport à la direction de sortie de la buse d'air comprimé (32).

8. Procédé conforme à la revendication 6 ou 7,
**caractérisé en ce que**
un autre flux d'air est respectivement transmis au moyen de buses d'air de soufflage (36) de l'émetteur optique et du récepteur optique vers la zone basse-pression, l'air comprimé étant de préférence respectivement transféré par une conduite de dérivation vers les buses d'air de soufflage (36).
